# EUROPÄISCHE PATENTSCHRIFT

(11) **EP 1 448 221 B1**
(45) Veröffentlichungstag und Bekanntmachung des Hinweises auf die Patenterteilung: **03.01.2007**
(21) Anmeldenummer: 02790384.8
(22) Anmeldetag: 15.11.2002
(51) Int. Cl.: A61K 38/09, A61K 9/08, A61K 47/26

(54) **INJEKTIONSLÖSUNG EINES LHRH-ANTAGONISTEN**
INJECTION SOLUTION COMPRISING AN LHRH ANTAGONIST
SOLUTION POUR INJECTION D'UN ANTAGONISTE LHRH

(30) Priorität: 26.11.2001 DE 10157628
(43) Veröffentlichungstag der Anmeldung: 25.08.2004
(73) Patentinhaber: Zentaris GmbH, 60314 Frankfurt/Main (DE)
(72) Erfinder: SARLIKIOTIS, Werner, GR-190 02 Peania (GR); BAUER, Horst, 91217 Hersbruck (DE); RISCHER, Matthias, 60388 Frankfurt (DE); ENGEL, Jürgen, 63755 Alzenau (DE); GÜTHLEIN, Frank, 79589 Binzen (DE); DI STEFANO, Dominique, 65479 Raunheim (DE)
(86) Internationale Anmeldenummer: PCT/EP2002/012798
(87) Internationale Veröffentlichungsnummer: WO 2003/045419

(56) Entgegenhaltungen:
- WO-A-01/21194
- WO-A-01/87265

## Beschreibung

Die Erfindung betrifft wässrige Injektionslösungen eines LHRH-Antagonisten unter Verwendung von Zusätzen von organischen, physiologisch verträglichen Säuren und/ oder Tensiden und deren Herstellung zur Verhinderung der Aggregation des LHRH-Antagonisten in Lösung. Die entsprechend der Erfindung hergestellten Injektionslösungen führen zusätzlich zu einer Erhöhung der Bioverfügbarkeit und ermöglichten die Verringerung des zu applizierenden Injektionsvolumens.

### Stand der Technik:

Bei der kontrollierten ovariellen Stimulation, gefolgt von einer Eizellentnahme und Techniken der assistierten Reproduktion werden neben LHRH-Agonisten (z.B. Triptorelin, Buserelin) vor allem seit einiger Zeit LHRH-Antagonisten (Cetrorelix, Ganirelix) verwendet, da sie den initialen Anstieg der endogenen Gonadotropinsekretion vermeiden und sofort zu einer kompetitiven Hemmung des Gonadotropin freisetzenden Hormons führen [EP 0 788 799 A2; EP 0 299 402 B1]. Der LHRH-Antagonist Ganirelix wird derzeit in einer Formulierung angewendet, die 0.25 mg Ganirelix in 0.5 ml einer wässrigen, mannitolhaltigen Lösung in Form einer Fertiginjektion enthält (Orgalutran^{®}). Der LHRH-Antagonist Cetrorelix (Cetrotide^{®}) wird derzeit in zwei Darreichungsformen angeboten: Ein Lyophilisat mit 0.25 mg Cetrorelix kombiniert mit einer Fertigspritze, die 1 ml Wasser zur Rekonstitution enthält sowie ein Lyophilisat mit 3 mg Cetrorelix kombiniert mit einer Fertigspritze, die 3 ml Wasser zur Rekonstitution enthält. LHRH-Antagonisten werden aber nicht nur zur kontrollierten ovariellen Stimulation verwendet, sondern können auch zur Therapie von hormonabhängigen Krebsarten wie z.B. dem Prostatakarzinom verwendet werden. Substanzen wie Abarelix [WO 98/25642] oder Cetrorelix [WO 00/47234] könnten dazu verwendet werden, daß die LHRH-Antagonisten eine Alternative zu den marktdominierenden Agonisten (Leuprolide, Goserelin) in dieser Therapie sein könnten. Aufgrund der relativ schlechten Löslichkeit von Abarelix in Wasser oder physiologischen Medien muß eine Depotformulierung verwendet werden, um eine Langzeitwirkung zu erreichen. Es gibt jedoch Hinweise, dass eine Langzeitwirkung auch durch eine gute Löslichkeit der LHRH-Antagonisten bedingt sein könnte [G. Jiang, J. Stakewski, R. Galyean, J. Dykert, C. Schteingart, P. Broqua, A. Aebi, M.L. Aubert, G. Semple, P. Robson, K. Akinsanya, R. Haigh, P. Riviere, J. Trojnar, J.L. Junien und J.E. Rivier, J. Med. Chem., 2001, 44, 453-467].

Aus der WO 01/87265 A2 ist eine pharmazeutische, zur parenteralen Anwendung geeignete Darreichungsform bekannt, die zur Aggregation neigende LHRH-Antagonisten in Form ihrer Acetat-, Gluconat-, Glucuronat-, Lactat-, Citrat-, Benzoat- oder Phosphat-Salze gelöst oder dispergiert enthält und zusätzlich eine der genannten Säuren als freie Säure umfasst.

### Darstellung der Erfindung

Aufgabe der Erfindung ist es, eine Injektionslösung herzustellen, die ein niedriges Injektionsvolumen bei einer erhöhten Konzentration des LHRH-Antagonisten durch dessen verbesserte Löslichkeit erreicht. Gleichzeitig soll die Aggregation des LHRH-Antagonisten in der höherkonzentrierten Injektionslösung verhindert werden.

Es wurde überraschenderweise gefunden, dass der Zusatz von Gluconsäuredeltalacton in Verbindung mit Tween 80 und Mannit die Löslichkeit von LHRH-Antagonisten deutlich verbessert und damit die Aggregationsneigung dieser Substanzen deutlich herabsetzt.

Die Erfindung macht es daher möglich, LHRH-Antagonisten in höherer Konzentration in wässrigen Lösungen zur Injektion bereitzustellen.

Die Tensid-Konzentration darf nicht zu hoch gewählt werden, da sonst die Lösungen zu sehr schäumen und wiederum Aggregation durch die Tenside induziert wird.

Der Gegenstand der Erfindung wird in den anhängenden Ansprüchen angegeben.

Gleichzeitig ermöglichen diese Zusätze eine Erhöhung der Bioverfügbarkeit, da sie offensichtlich auch die spontane Aggregation im Körper nach Injektion verlangsamen bzw. eine schnellere Resorption der Substanz vom Wirkort ermöglichen. Es zeigte sich, daß der erniedrigte pH-Wert solcher Injektionslösungen (z.B. pH= 2.5 - 3) keinen Einfluß auf die lokale Verträglichkeit der Injektion hat. Durch die Erhöhung der Konzentration ist es möglich, das applizierte Volumen zu reduzieren, z.B. bei Cetrorelix für die 3 mg Form von 3 ml auf 1 ml. Es wurde ebenfalls gezeigt, daß durch diese Zusätze eine gute Lagerstabilität erreicht werden kann (s. Beispiel 1). Eine Lagerung über 6 Monate bei 25°C/ 60% ergab zwar einen Anstieg der Verunreinigungen, aber der Gehaltswert lag in jedem Fall noch deutlich über 90% (in der Regel niedrigster Wert der Laufzeitspezifikation pharmazeutischer Produkte). Die Trübung, als Anzeichen für Aggregation, nahm nur wenig zu. Trübungswerte bis zu 8 FTU (formazin turbidity unit nach Pharmacopoea Europaea) sind durchaus tolerierbar.

Konservierungsmittel wie z.B. Phenol oder p-Chlor-m-Kresol stören nicht und können zusätzlich zur Konservierung der Lösungen verwendet werden.

### Beschreibung eines Weges zur Ausführung der Erfindung:

### Beispiel 1

| | |
|---|---|
| 500 mg | Cetrorelix |
| 2 g | Tween 80 |
| 2.4g | Gluconsäuredeltalacton |
| 95g | Mannit |

wurden mit Wasser für Injektionszwecke ad 2 Liter zu einer homogenen Lösung gemischt. Die Lösung wurde anschließend steril filtriert und in Ampullen abgefüllt. Die Ampullen wurden initial und nach 6 Monaten Lagerzeit bei 2-8°C und 25°C/ 60% r.F. auf Reinheit (HPLC), Gehalt (HPLC), pH-Wert und Aggregation (Trübung) analytisch untersucht.

### Analytische Ergebnisse:

| | Ausgangs -untersuchung | Untersuchung nach 6 Monaten bei 2-8°C | Untersuchung nach 6 Monaten bei 25°C/ 60% r.F. |
|---|---|---|---|
| Reinheit [%] | 0.37 | 0.69 | 2.32 |
| Gehalt [%] | 100. 0 | 98.7 | 95.4 |
| pH-Wert | 3.12 | 3.16 | 3.16 |
| Trübung [FTU] | 1.88 | 2.62 | 3.92 |

### Beispiel 2

Ca. 500 mg D-63153
Ca. 100 mg Tween 80
Ca. 475 mg Mannit
wurden mit wässriger, gesättigter Gluconsäure-deltalacton-lösung auf einen pH-Wert von ca. 2.5 eingestellt. Es resultierte ein Volumen von ca. 50 ml. Es wurde solange gerührt bis eine klare Lösung resultierte.

### Analytische Ergebnisse:

Die Trübung der Lösung betrug anfangs 2.4 FTU. Nach 24 h wurden 2.1 FTU gemessen. Das Reinheitsprofil und der Gehalt der Lösung (HPLC) blieben unverändert.

### Struktur des LHRH-Antagonisten D-63153:

Ac-D-Nal-D-pCl-Phe-D-Pal-Ser-N-Me-Tyr-D-Hci-Nle-Arg-Pro-D-Ala-NH2

### Beispiel 3

Ca. 100 mg Teverelix
Ca. 100 mg Tween 80
Ca. 475 mg Mannit
wurden mit wässriger, gesättigter Gluconsäure-deltalacton-lösung auf einen pH-Wert von ca. 2.5 eingestellt. Es resultierte ein Volumen von ca. 10 ml. Es wurde solange gerührt bis eine klare Lösung resultierte.

### Analytische Ergebnisse:

Die Trübung der Lösung betrug anfangs 6.8 FTU. Nach 24 h wurden 8.4 FTU gemessen. Das Reinheitsprofil und der Gehalt der Lösung (HPLC) blieben unverändert.

### Struktur des LHRH-Antagonisten Teverelix:

Ac-D-Nal-pCl-D-Phe-3-D-Pal-Ser-Tyr-D-H-Cit- Leu-iPr-Lys-Pro-D-Ala-NH2

## Patentansprüche

1. Wässrige Injektionslösung eines LHRH-Antagonisten enthaltend Gluconsäure, ein Tensid sowie einen Gerüstbildner, **dadurch gekennzeichnet, dass** als LHRH-Antagonist Cetrorelix, Teverelix, D-63 153 (Ac-D-Nal-pCl-D-Phe- 3-D-Pal-Ser-N-Me-Tyr-D-H-Cit-Iso-Leu-Arg-Pro-D-Ala-NH2), Ganirelix, Abarelix, Antide, oder Azaline B eingesetzt wird, und **dass** die Gluconsäure in mehr als einer äquimolaren Menge bezogen auf die Menge des LHRH-Antagonisten vorliegt, dass die Gluconsäure in Form von Gluconsäuredeltalacton zugesetzt wird, dass als Tensid Tween 80 und als Gerüstbildner Mannit eingesetzt wird.

2. Wässrige Injektionslösung eines LHRH-Antagonisten nach Anspruch 1, enthaltend
500 mg Cetrorelix
2,4 g Gluconsäuredeltalacton
2,0 g Tween 80
95,0 g Mannit
in 2 l Wasser ad injectionem.

3. Wässrige Injektionslösung eines LHRH-Antagonisten nach Anspruch 1, enthaltend
500 mg D-63 153
100 mg Tween 80
475 mg Mannit
mit gesättigter Gluconsäuredeltalactonlösung auf 50 ml eingestellt.

4. Wässrige Injektionslösung eines LHRH-Antagonisten nach Anspruch 1, enthaltend
100 mg Teverelix
100 mg Tween 80
475 mg Mannit
mit gesättigter Gluconsäuredeltalactonlösung auf 10 ml eingestellt.

5. Verfahren zur Herstellung wässriger Injektionslösungen eines LHRH-Antagonisten gemäß einem der Ansprüche 1 bis 4, **gekennzeichnet dadurch, dass** alternativ
- ein LHRH-Antagonist, Gluconsäure in Form von Gluconsäuredeltalacton, wobei die Gluconsäure in mehr als einer äquimolaren Menge bezogen auf die Menge des LHRH-Antagonisten vorliegt, Tween 80 als Tensid und Mannit als Gerüstbildner in Wasser ad injectionem gelöst, homogenisiert und für Injektionszwecke verarbeitet werden, oder
- ein LHRH-Antagonist, Tween 80 als Tensid und Mannit als Gerüstbildner in wässriger gesättigter Lösung von Gluconsäuredeltalacton gelöst, homogenisiert und für Injektionszwecke verarbeitet werden.

## Claims

1. Aqueous injection solution of an LHRH antagonist containing gluconic acid, a surfactant and a bulking agent, **characterized in that** cetrorelix, teverelix, D-63 153 (Ac-D-Nal-pCl-D-Phe-3-D-Pal-Ser-N-Me-Tyr-D-H-Cit-Iso-Leu-Arg-Pro-D-Ala-NH2), ganirelix, abarelix, antide or azaline B is used as LHRH antagonist and **in that** the gluconic acid is present in a more than equimolar amount, based on the amount of the LHRH antagonist, **in that** the gluconic acid is added in the form of gluconic acid delta-lactone, and **in that** Tween 80 is used as surfactant and mannitol is used as bulking agent.

2. Aqueous injection solution of an LHRH antagonist according to Claim 1, comprising
500 mg of cetrorelix
2.4 g of gluconic acid delta-lactone
2.0 g of Tween 80
95.0 g of mannitol
in 2 l of water for injection.

3. Aqueous injection solution of an LHRH antagonist according to Claim 1, comprising
500 mg of D-63 153
100 mg of Tween 80
475 mg of mannitol
adjusted to 50 ml using saturated gluconic acid delta-lactone solution.

4. Aqueous injection solution of an LHRH antagonist according to Claim 1, comprising
100 mg of teverelix
100 mg of Tween 80
475 mg of mannitol
adjusted to 10 ml using saturated gluconic acid delta-lactone solution.

5. Process for the preparation of aqueous injection solutions of an LHRH antagonist according to any one of Claims 1 to 4, **characterized in that**, alternatively
- an LHRH antagonist, gluconic acid in the form of gluconic acid delta-lactone, the gluconic acid being present in a more than equimolar amount based on the amount of LHRH antagonist, Tween 80 as surfactant and mannitol as bulking agent are dissolved in water for injection, homogenized and processed for injection purposes, or
- an LHRH antagonist, Tween 80 as surfactant and mannitol as bulking agent are dissolved in an aqueous saturated solution of gluconic acid delta-lactone, homogenized and processed for injection purposes.

## Revendications

1. Solution aqueuse pour injection d'antagonistes LHRH contenant de l'acide gluconique, un tensioactif ainsi qu'un agent d'édification du squelette,
**caractérisée en ce que**
comme antagoniste LHRH on utilise le Cetrorelix, le Teverelix, le D-63 153 (Ac-D-Nal-pCl-D-Phe-3-D-Pal-Ser-N-Me-Tyr-D-H-Cit-Iso-Leu-Arg-Pro-D-Ala-NH2), le Ganirelix, l'Abarelix, l'Antide ou l'Azaline B, et l'acide gluconique est présent en une quantité plus qu'équimolaire par rapport à la quantité de l'antagoniste LHRH, l'acide gluconique est ajouté sous la forme de delta-lactone d'acide gluconique, et on utilise comme tensioactif le Tween 80 et comme agent d'édification du squelette le Mannitol.

2. Solution aqueuse pour injection d'antagonistes LHRH selon la revendication 1, contenant :
500 mg de Cetrorelix
2,4 g de delta-lactone d'acide gluconique
2,0 g de Tween 80
95,0 g de Mannitol
dans 2 l d'eau ad injectionem.

3. Solution aqueuse pour injection d'antagonistes LHRH selon la revendication 1, contenant :
500 mg de D-63 153
100 mg de Tween 80
475 mg de Mannitol
ajusté à 50 ml par une solution saturée de delta-lactone d'acide gluconique.

4. Solution aqueuse pour injection d'antagonistes LHRH selon la revendication 1, contenant :
100 mg de Teverelix
100 mg de Tween 80
475 mg de Mannitol
ajusté à 10 ml par une solution saturée de delta-lactone d'acide gluconique.

5. Procédé de préparation de solutions aqueuses pour injection d'antagonistes LHRH selon l'une des revendications 1 à 4,
**caractérisé en ce que**
- on dilue dans de l'eau ad injectionem, on homogénéise et on travaille en vue d'une injection ultérieure un antagoniste LHRH, de l'acide gluconique sous la forme de delta-lactone d'acide gluconique, l'acide gluconique étant présent en une quantité plus qu'équimolaire par rapport à la quantité de l'antagoniste LHRH, du Tween 80 à titre de tensioactif et du Mannitol à titre d'agent d'édification du squelette, soit
- on dilue dans une solution saturée de delta-lactone d'acide gluconique, on homogénéise et on travaille en vue d'une injection ultérieure un antagoniste LHRH, du Tween 80 à titre de tensioactif et du Mannitol à titre d'agent d'édification du squelette.
